(19) **Europäisches Patentamt**
**European Patent Office**
**Office européen des brevets**

(11) **EP 4 287 121 A1**

(12) **EUROPEAN PATENT APPLICATION**
published in accordance with Art. 153(4) EPC

(43) Date of publication:
**06.12.2023 Bulletin 2023/49**

(21) Application number: **22746153.0**

(22) Date of filing: **20.01.2022**

(51) International Patent Classification (IPC):
*G06T 7/33* (2017.01)    *G06T 11/00* (2006.01)
*G06T 15/20* (2011.01)    *A61B 6/00* (2006.01)
*A61B 6/14* (2006.01)    *A61B 6/03* (2006.01)
*A61B 5/00* (2006.01)    *A61C 9/00* (2006.01)

(86) International application number:
**PCT/KR2022/001042**

(87) International publication number:
**WO 2022/164126 (04.08.2022 Gazette 2022/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.01.2021 KR 20210011835**

(71) Applicant: **HDX Will Corp.**
**Seoul 03162 (KR)**

(72) Inventors:
• **JUNG, Hong**
**Seoul 03162 (KR)**
• **LEE, Sung Min**
**Bucheon-si Gyeonggi-do 14553 (KR)**
• **SONG, Bo Hyun**
**Seoul 06723 (KR)**

(74) Representative: **BOVARD AG**
**Patent- und Markenanwälte**
**Optingenstrasse 16**
**3013 Bern (CH)**

(54) **DEVICE AND METHOD FOR AUTOMATICALLY MATCHING ORAL SCAN DATA AND COMPUTED TOMOGRAPHY IMAGE BY MEANS OF CROWN SEGMENTATION OF ORAL SCAN DATA**

(57) Disclosed is a device, for matching oral scan data, comprising a first matching unit, a teeth segmentation unit, and a second matching unit. The first matching unit matches a coordinate system of three-dimensional oral scan data to a coordinate system of a three-dimensional oral computed tomography (CT) image by using three-dimensional feature points of the three-dimensional oral scan data and three-dimensional feature points of the three-dimensional oral CT image. The teeth segmentation unit segments teeth from the three-dimensional oral scan data on the basis of the three-dimensional feature points of the three-dimensional oral scan data to generate surface information relating to the segmented teeth. The second matching unit matches the three-dimensional oral scan data and the three-dimensional oral CT image, which have matching coordinate systems, by means of the surface information.

FIG. 2

# Description

[Technical Field]

The present invention relates to a device and method for matching intraoral scan data and a computed tomography (CT) image.

[Background Art]

In the dental field including orthodontics and implants, a computed tomography (CT) image and scan data for the oral cavity are widely used for establishing diagnosis plans or undergoing treatment. A CT image has an advantage of being able to show dental root information, but image resolution is low, and the accuracy of the image is very low due to various artifacts. On the other hand, scan data has an advantage of having higher tooth crown precision than the CT image and no artifacts, but dental root information is not shown. Accordingly, an intraoral model in which the CT image and the scan data are matched is widely used.

However, when intraoral scan data and a CT image are matched, a geometric difference occurs between the intraoral scan data and the CT image.

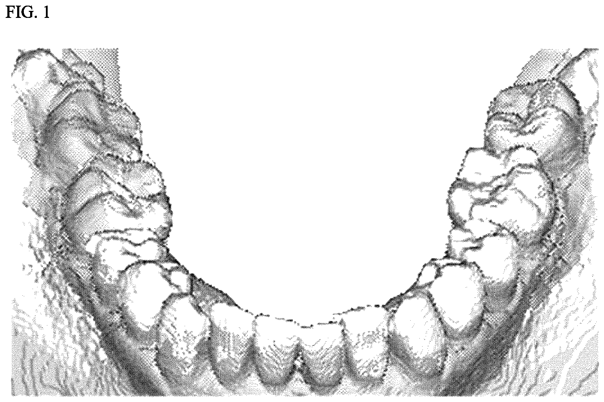

FIG. 1 shows an example of matching errors occurring between scan data and a CT image.

Since intraoral scan data is an image reconstructed by matching several pieces of data obtained by locally photographing the entire oral cavity, there are accumulated errors, and thus a geometric difference between the intraoral scan data and a CT image occurs when the intraoral scan data and the CT image are matched.

[Related Art Document]

[Patent Document]

(Patent Document 1) Korean Patent Registration No. 10-1911327

[Disclosure]

[Technical Problem]

The present invention is directed to providing a method and device for matching three-dimensional (3D) intraoral scan data that can reduce errors occurring when scan data and a computed tomography (CT) image are matched.

[Technical Solution]

One aspect of the present invention provides a method of matching intraoral scan data, which includes matching a coordinate system of three-dimensional (3D) intraoral scan data to a coordinate system of a 3D intraoral computed tomography (CT) image using 3D feature points of the 3D intraoral scan data and 3D feature points of the 3D intraoral CT image; segmenting teeth in the 3D intraoral scan data on the basis of the 3D feature points of the 3D intraoral scan data and generating surface information about the segmented teeth; and matching the 3D intraoral scan data and the 3D intraoral CT image, whose coordinate systems have been matched, using the surface information.

The method of matching intraoral scan data may further include determining the 3D feature points of the 3D intraoral scan data.

The determining of the 3D feature points of the 3D intraoral scan data may include performing rendering on the 3D intraoral scan data and generating two-dimensional (2D) intraoral scan data; determining 2D feature points of the 2D intraoral scan data; and projecting the 2D feature points of the 2D intraoral scan data onto the 3D intraoral scan data and determining the 3D feature points of the 3D intraoral scan data.

The determining of the 2D feature points of the 2D intraoral scan data may include applying deep learning to the 2D intraoral scan data and generating a 2D heatmap representing adjacent points between teeth in the 2D intraoral scan data; and determining the 2D feature points of the 2D intraoral scan data using the 2D heatmap.

The method of matching intraoral scan data may further include determining the 3D feature points of the 3D intraoral CT image.

The determining of the 3D feature points of the 3D intraoral CT image may include performing rendering on the 3D intraoral CT image and generating a 2D intraoral CT image; determining 2D feature points of the 2D intraoral CT image; and projecting the 2D feature points of the 2D intraoral CT image onto the 3D intraoral CT image and determining the 3D feature points of the 3D intraoral CT image.

The determining of the 2D feature points of the 2D intraoral CT image may include applying deep learning to the 2D intraoral CT image and generating a 2D heatmap representing adjacent points between teeth in the 2D intraoral CT image; and determining the 2D feature points of the 2D intraoral CT image using the 2D heatmap.

The generating of the surface information about the segmented teeth may include generating the surface information about the segmented teeth by segmenting the teeth from the 3D intraoral scan data through a harmonic function in which the 3D feature points of the 3D scan data are used as a boundary condition.

The harmonic function may be a function that gives weights to concave points of the 3D scan data.

The method of matching intraoral scan data may further include acquiring the 3D intraoral scan data from an intraoral scanner; and acquiring the 3D intraoral CT image from CT equipment.

Another aspect of the present invention provides a device for matching intraoral scan data, which include a first matching unit configured to match a coordinate system of 3D intraoral scan data to a coordinate system of 3D intraoral CT image using 3D feature points of the 3D intraoral scan data and 3D feature points of the 3D intraoral CT image; a teeth segmentation unit configured to segment teeth in the 3D intraoral scan data on the basis of the 3D feature points of the 3D intraoral scan data to generate surface information about the segmented teeth; and a second matching unit configured to match the 3D intraoral scan data and the 3D intraoral CT image, whose coordinate systems have been matched, using the surface information.

The device for matching intraoral scan data may further include a scan data feature point determination unit configured to project 2D feature points of 2D intraoral scan data generated from the 3D intraoral scan data onto the 3D intraoral scan data to determine the 3D feature points of the 3D intraoral scan data.

The method of matching intraoral scan data may further include a CT image feature point determination unit configured to project 2D feature points of the 2D intraoral CT image generated from the 3D intraoral CT image onto the 3D intraoral CT image to determine the 3D feature points of the 3D intraoral CT image.

Still another aspect of the present invention provides an intraoral scanner which includes the device for matching intraoral scan data.

Yet another aspect of the present invention provides a CT device which includes the device for matching intraoral scan data.

Yet another aspect of the present invention provides a computer program stored in a computer-readable medium, wherein, when a command of the computer program is executed, the method of matching intraoral scan data is performed.

[Advantageous Effects]

According to the embodiment of the present invention, by individually segmenting teeth in intraoral scan data and then matching each of the segmented teeth and a computed tomography (CT) image, it is possible to reduce errors occurring when the intraoral scan data and the segmented teeth are matched.

[Description of Drawings]

FIG. 1 shows an example of matching errors occurring between scan data and a computed tomography (CT) image.
FIG. 2 is a block diagram of a device for matching three-dimensional (3D) intraoral scan data according to an embodiment of the present invention.
FIG. 3 is a flowchart illustrating operations of a device for matching 3D intraoral scan data according to an embodiment of the present invention.
FIG. 4 shows a method of detecting 3D feature points using deep learning according to an embodiment of the present invention.
FIG. 5 shows matching of 3D scan data and a 3D CT image according to an embodiment of the present invention.
FIG. 6 shows the generation of surface information about a tooth according to an embodiment of the present invention.
FIG. 7 shows the crown segmentation of teeth in scan data according to an embodiment of the present invention.
FIG. 8 shows the matching of 3D scan data and a 3D CT image through tooth crown surface matching according to an embodiment of the present invention.
FIG. 9 shows a process of automatically matching intraoral scan data and a CT image using crown segmentation of intraoral scan data according to an embodiment of the present invention.

[Modes of the Invention]

Hereinafter, embodiments of the present invention that can be easily performed by those skilled in the art will be described in detail with reference to the accompanying drawings. In addition, parts irrelevant to description are omitted in the drawings in order to clearly explain the present invention.

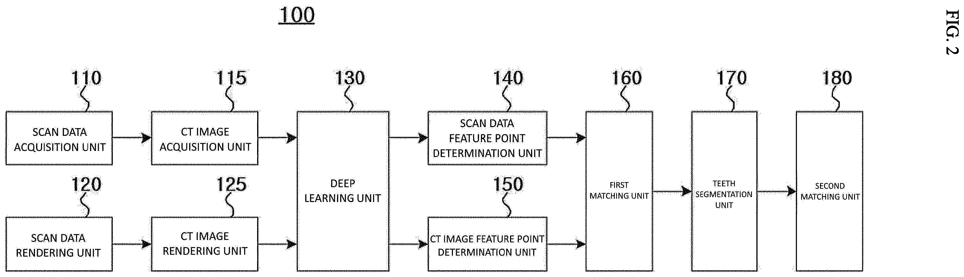

Next, a device for matching three-dimensional (3D) intraoral scan data according to an embodiment of the present invention will be described with reference to FIG. 2.

FIG. 2 is a block diagram of a device for matching 3D intraoral scan data according to an embodiment of the present invention.

As illustrated in FIG. 2, a device 100 for matching 3D intraoral scan data according to the embodiment of the present invention includes a scan data acquisition unit 110, a scan data rendering unit 115, a computed tomography (CT) image acquisition unit 120, a CT image rendering unit 125, a deep learning unit 130, a scan data feature point determination unit 140, a CT image feature point determination unit 150, a first matching unit 160, a teeth segmentation unit 170, and a second matching unit 180.

The operation of each component of the device 100 for matching 3D intraoral scan data will be described with reference to the following drawings.

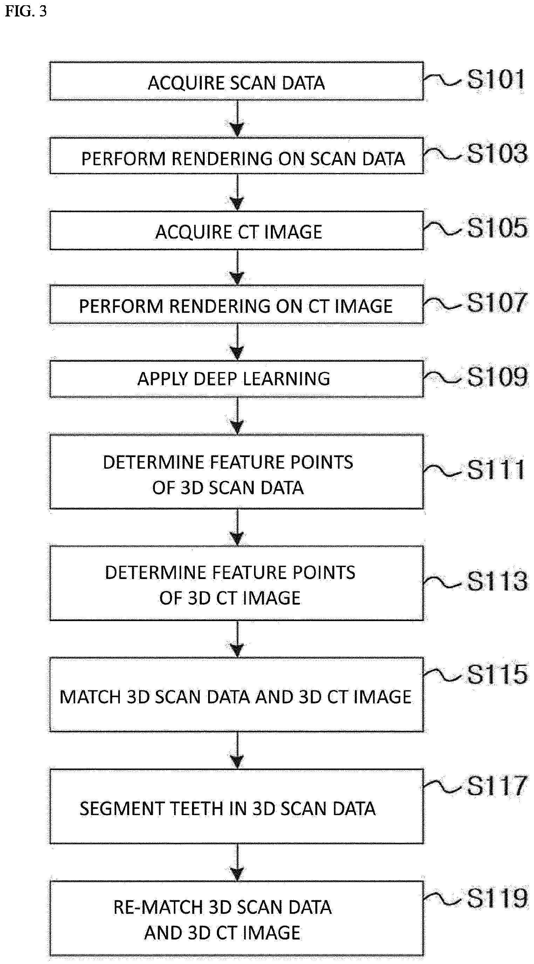

FIG. 3 is a flowchart illustrating the operations of the device for matching 3D intraoral scan data according to the embodiment of the present invention.

The scan data acquisition unit 110 acquires 3D scan data for an oral cavity of a patient using an intraoral scanner (S101).

The scan data rendering unit 115 generates two-dimensional (2D) scan data by performing 2D rendering on the 3D scan data in a z direction (S103).

The CT image acquisition unit 120 acquires a 3D CT image of the oral cavity of the patient from CT equipment (S105).

The CT image rendering unit 125 generates a 2D CT image by performing 2D rendering on the 3D CT image in the z direction (S107).

The deep learning unit 130 applies deep learning to the 2D scan data and the 2D CT image to generate a 2D heatmap representing adjacent points between teeth in the 2D scan data and a 2D heatmap representing adjacent points between teeth in the 2D CT image (S109). A heatmap is a data visualization technique that shows the intensity of a phenomenon as color in two dimensions. Therefore, the deep learning unit 130 may perform deep learning and output a heatmap that shows the degree of proximity of adjacent points between teeth as color.

The scan data feature point determination unit 140 determines 2D feature points of the 2D scan data using the 2D heatmap representing the adjacent points between teeth in the 2D scan data, and projects the determined 2D feature points onto the 3D scan data to determine 3D feature points of the 3D scan data (S111).

The CT image feature point determination unit 150 determines 2D feature points of the 2D CT image using the 2D heatmap representing the adjacent points between teeth in the 2D CT image, and projects the determined 2D feature points onto the 3D CT image to determine 3D feature points of the 3D CT image (S113).

The first matching unit 160 applies an iterative surface matching-based algorithm using the 3D feature points of the 3D scan data and the 3D feature points of the 3D CT image as initial values, and matches a coordinate system of the 3D scan data to a coordinate system of the 3D CT image (S115). In one embodiment, the first matching unit 160 may obtain a rigid-body transformation matrix T that minimizes an error E(T) shown in Equation 1, and match the coordinate system of the 3D scan data to the coordinate system of the 3D CT image.

[Equation 1]

$$E(T) = \left\| C - T(S) \right\|^2$$

Here, C denotes a point cloud of CT data, and S denotes a point cloud of scan data.

The teeth segmentation unit 170 segments the teeth into a plurality of teeth, in the 3D scan data whose coordinate system matches the coordinate system of the 3D CT image, on the basis of the 3D feature points of the 3D scan data to generate surface information about the plurality of segmented teeth (S1 17). In one embodiment, the teeth segmentation unit 170 may segment the teeth by solving a weighted harmonic function in which the 3D feature points of the 3D scan data are used as a boundary condition to give weights to concave points of the 3D scan data, which is oral cavity scan mesh data. Conventionally, the user manually inputs the boundary condition to the weighted harmonic function, but in the embodiment of the present invention, the 3D feature points of the 3D scan data are used in the weighted harmonic function, and thus, according to the present invention, the teeth may be easily and automatically segmented without user intervention. Specifically, the teeth segmentation unit 170 may segment the teeth by solving the following weighted harmonic function ΔΦ=0.

A harmonic field on a 3D manifold triangular mesh M=(V, E, F) satisfies ΔΦ=0 as a scalar field belonging to each mesh point. Here, V denotes a set of vertices of M, E denotes a set of edges of M, and F denotes a set of faces of M. Δ is a Laplacian operator subject to the Dirichlet boundary condition. In this case, the 3D feature points of the 3D scan data may be used as the Dirichlet boundary condition.

A standard definition of the Laplace operator on a piecewise linear mesh M is an umbrella operator as shown in Equation 2.

[Equation 2]

$$\Delta \Phi_i = \sum_{(i,j) \in E} w_{ij} (\Phi_i - \Phi_j)$$

Here, $w_{ij}$ denotes a scalar weight assigned to an edge $E_{ij}$.

The second matching unit 180 applies an iterative surface matching-based algorithm to the region of the segmented teeth to re-match the 3D scan data and the 3D CT image, whose coordinate systems have been matched (S119). In one embodiment, the second matching unit 180 may apply an iterative surface matching-based algorithm on the basis of the surface information of each of the plurality of segmented teeth to re-match the 3D scan data and the 3D CT image, whose coordinate systems have been matched.

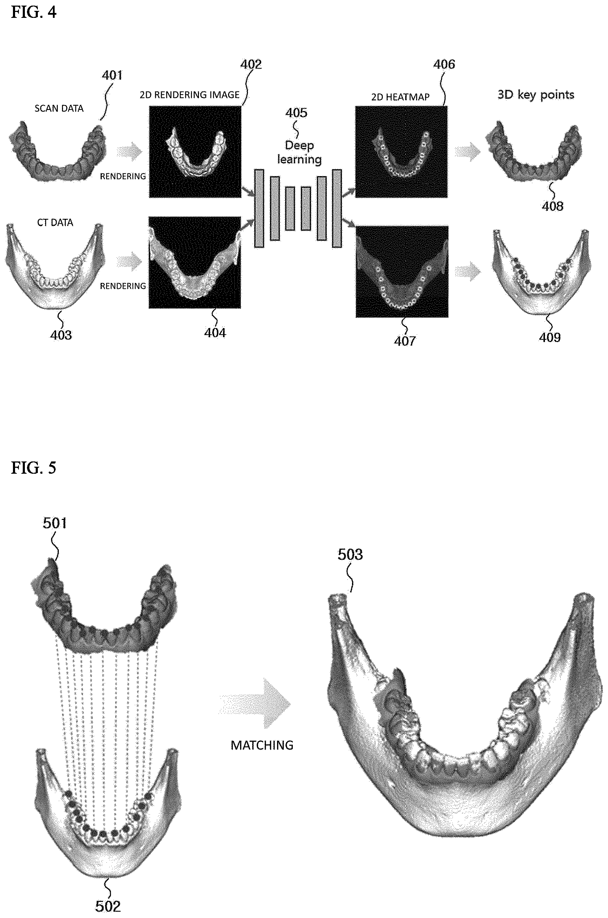

FIG. 4 shows a method of detecting 3D feature points using deep learning according to an embodiment of the present invention.

As shown in FIG. 4, 3D scan data 401 of an oral cavity of a patient is acquired from an intraoral scanner, and 2D rendering is performed on the acquired 3D scan data in a z direction to generate 2D scan data 402. A 3D CT image 403 of the oral cavity of the patient is acquired from CT equipment, and 2D rendering is performed on the acquired 3D CT image in the z direction to generate a 2D CT image 404.

Deep learning is applied to the 2D scan data 402 and the 2D CT image 404, and a 2D heatmap 406 representing adjacent points between teeth in 2D scan data and a 2D heatmap 407 representing adjacent points between teeth in a 2D CT image are generated.

Thereafter, according to the embodiment of the present invention, 3D feature points 408 of the 3D scan data may be determined using the 2D heatmap 406 of the 2D scan data, and 3D feature points 409 of the 3D CT image may be determined using the 2D heatmap 407 of the 2D CT image.

FIG. 5 shows matching of 3D scan data and a 3D CT image according to an embodiment of the present invention.

As shown in FIG. 5, according to the embodiment of the present invention, the 3D scan data and the 3D CT image may be matched (503) using 3D feature points 501 of the 3D scan data and 3D feature points 502 of the 3D CT image.

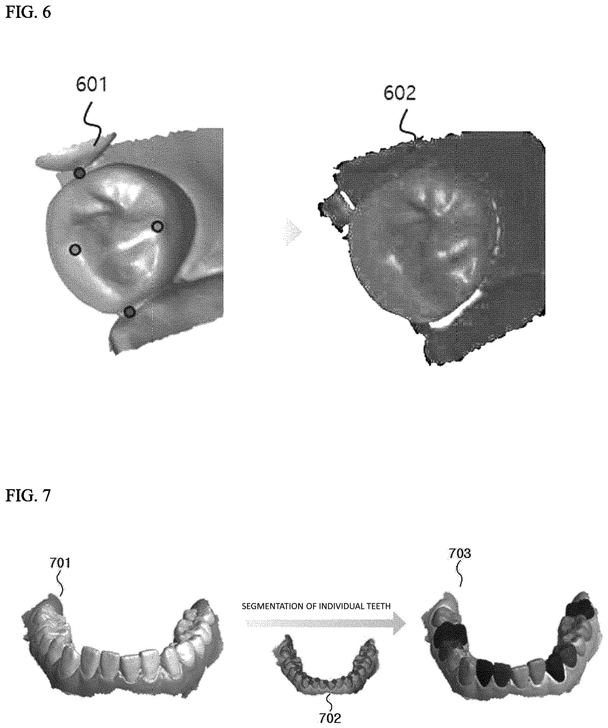

FIG. 6 shows the generation of surface information about a tooth according to an embodiment of the present invention.

As shown in FIG. 6, surface information 602 about the segmented tooth may be generated based on 3D feature points 601 of 3D scan data.

FIG. 7 shows the crown segmentation of teeth in scan data according to an embodiment of the present invention.

As shown in FIG. 7, a tooth crown may be segmented (703) from 3D scan data 701 on the basis of 3D feature points 702 of the 3D scan data.

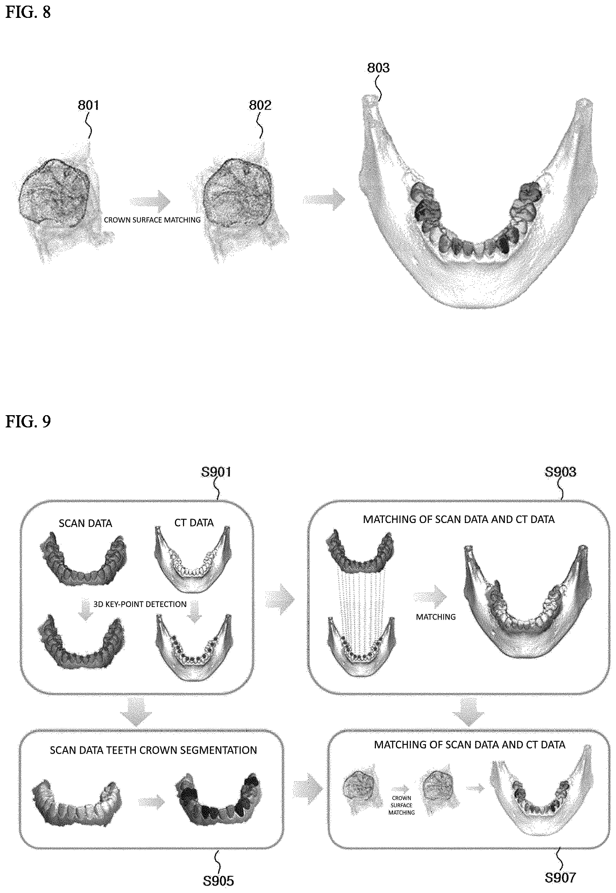

FIG. 8 shows the matching of 3D scan data and a 3D CT image through tooth crown surface matching according to an embodiment of the present invention.

As shown in FIG. 8, a tooth segmented from scan data may be matched with a CT image through crown surface matching. A crown surface 801 that does not match the CT image may be matched with the CT image (802) through surface matching, and the 3D scan data and the 3D CT image may be matched (803) by repeatedly applying such matching to a plurality of teeth.

FIG. 9 shows a process of automatically matching intraoral scan data and a CT image using crown segmentation of intraoral scan data according to an embodiment of the present invention.

As shown in FIG. 9, according to an embodiment of the present invention, first, 3D feature points of the scan data may be determined from the scan data, and 3D feature points of the CT image may be determined from the CT image (S901).

Thereafter, the 3D scan data and the 3D CT image may be matched using the 3D feature points of the 3D scan data and the 3D feature points of the 3D CT image (S903).

A tooth crown may be segmented from the 3D scan data on the basis of the 3D feature points of the 3D scan data (S905).

Next, a tooth segmented from the scan data may be matched with the CT image through crown surface matching (S907).

The device for matching 3D intraoral scan data according to the embodiment of the present invention may be included as a module in a CT device or an intraoral scanner, or may be implemented in software in which CT data and/or intraoral scanner data are used.

While the present invention has been described with reference to the embodiment illustrated in the accompanying drawings, the embodiment should be considered in a descriptive sense only, and it should be understood by those skilled in the art that various alterations and equivalent other embodiments may be made. Therefore, the scope of the present invention should be defined by only the following claims.

**Claims**

1. A method of matching intraoral scan data, comprising:

   matching a coordinate system of three-dimensional (3D) intraoral scan data to a coordinate system of a 3D intraoral computed tomography (CT) image using 3D feature points of the 3D intraoral scan data and 3D feature points of the 3D intraoral CT image;
   segmenting teeth in the 3D intraoral scan data on the basis of the 3D feature points of the 3D intraoral scan data and generating surface information about the segmented teeth; and
   matching the 3D intraoral scan data and the 3D intraoral CT image, whose coordinate systems have been matched, using the surface information.

2. The method of claim 1, further comprising determining the 3D feature points of the 3D intraoral scan data.

3. The method of claim 2, wherein the determining of the 3D feature points of the 3D intraoral scan data includes:

   performing rendering on the 3D intraoral scan data and generating two-dimensional (2D) intraoral scan data;
   determining 2D feature points of the 2D intraoral scan data; and
   projecting the 2D feature points of the 2D intraoral scan data onto the 3D intraoral scan data and determining the 3D feature points of the 3D intraoral scan data.

4. The method of claim 3, wherein the determining of the 2D feature points of the 2D intraoral scan data includes:

   applying deep learning to the 2D intraoral scan data and generating a 2D heatmap representing adjacent points between teeth in the 2D intraoral scan data; and
   determining the 2D feature points of the 2D intraoral scan data using the 2D heatmap.

5. The method of claim 1, further comprising determining the 3D feature points of the 3D intraoral CT image.

6. The method of claim 5, wherein the determining of the 3D feature points of the 3D intraoral CT image includes:

   performing rendering on the 3D intraoral CT image and generating a 2D intraoral CT image; determining 2D feature points of the 2D intraoral CT image; and projecting the 2D feature points of the 2D intraoral CT image onto the 3D intraoral CT image and determining the 3D feature points of the 3D intraoral CT image.

7. The method of claim 6, wherein the determining of the 2D feature points of the 2D intraoral CT image includes:

   applying deep learning to the 2D intraoral CT image and generating a 2D heatmap representing adjacent points between teeth in the 2D intraoral CT image; and determining the 2D feature points of the 2D intraoral CT image using the 2D heatmap.

8. The method of claim 1, wherein the generating of the surface information about the segmented teeth includes generating the surface information about the segmented teeth by segmenting the teeth from the 3D intraoral scan data through a harmonic function in which the 3D feature points of the 3D scan data are used as a boundary condition.

9. The method of claim 8, wherein the harmonic function is a function that gives weights to concave points of the 3D scan data.

10. The method of claim 1, further comprising:

    acquiring the 3D intraoral scan data from an intraoral scanner; and acquiring the 3D intraoral CT image from CT equipment.

11. A device for matching intraoral scan data, comprising:

    a first matching unit configured to match a coordinate system of three-dimensional (3D) intraoral scan data to a coordinate system of 3D intraoral computed tomography (CT) image using 3D feature points of the 3D intraoral scan data and 3D feature points of the 3D intraoral CT image;
a teeth segmentation unit configured to segment teeth in the 3D intraoral scan data on the basis of the 3D feature points of the 3D intraoral scan data to generate surface information about the segmented teeth; and
a second matching unit configured to match the 3D intraoral scan data and the 3D intraoral CT image, whose coordinate systems have been matched, using the surface information.

12. The device of claim 11, further comprising a scan data feature point determination unit configured to project two-dimensional (2D) feature points of 2D intraoral scan data generated from the 3D intraoral scan data onto the 3D intraoral scan data to determine the 3D feature points of the 3D intraoral scan data.

13. The device of claim 11, further comprising a CT image feature point determination unit configured to project 2D feature points of the 2D intraoral CT image generated from the 3D intraoral CT image onto the 3D intraoral CT image to determine the 3D feature points of the 3D intraoral CT image.

14. An intraoral scanner comprising the device for matching intraoral scan data according to any one claim of claims 11 to 13.

15. A computed tomography (CT) device comprising the device for matching intraoral scan data according to any one claim of claims 11 to 13.

16. A computer program stored in a computer-readable medium, wherein, when a command of the computer program is executed, the method according to any one claim of claims 1 to 10 is performed.

FIG. 1

FIG. 2

100

| 110 SCAN DATA ACQUISITION UNIT | → | 115 CT IMAGE ACQUISITION UNIT | → | 130 DEEP LEARNING UNIT | → | 140 SCAN DATA FEATURE POINT DETERMINATION UNIT | → | 160 FIRST MATCHING UNIT | → | 170 TEETH SEGMENTATION UNIT | → | 180 SECOND MATCHING UNIT |

120 SCAN DATA RENDERING UNIT → 125 CT IMAGE RENDERING UNIT → 130 DEEP LEARNING UNIT → 150 CT IMAGE FEATURE POINT DETERMINATION UNIT → 160 FIRST MATCHING UNIT

FIG. 3

```
┌─────────────────────────────────┐
│       ACQUIRE SCAN DATA          │～S101
└─────────────────────────────────┘
              ↓
┌─────────────────────────────────┐
│   PERFORM RENDERING ON SCAN DATA │～S103
└─────────────────────────────────┘
              ↓
┌─────────────────────────────────┐
│        ACQUIRE CT IMAGE          │～S105
└─────────────────────────────────┘
              ↓
┌─────────────────────────────────┐
│    PERFORM RENDERING ON CT IMAGE │～S107
└─────────────────────────────────┘
              ↓
┌─────────────────────────────────┐
│        APPLY DEEP LEARNING       │～S109
└─────────────────────────────────┘
              ↓
┌─────────────────────────────────┐
│     DETERMINE FEATURE POINTS     │～S111
│         OF 3D SCAN DATA          │
└─────────────────────────────────┘
              ↓
┌─────────────────────────────────┐
│     DETERMINE FEATURE POINTS     │～S113
│          OF 3D CT IMAGE          │
└─────────────────────────────────┘
              ↓
┌─────────────────────────────────┐
│  MATCH 3D SCAN DATA AND 3D CT IMAGE │～S115
└─────────────────────────────────┘
              ↓
┌─────────────────────────────────┐
│    SEGMENT TEETH IN 3D SCAN DATA │～S117
└─────────────────────────────────┘
              ↓
┌─────────────────────────────────┐
│       RE-MATCH 3D SCAN DATA      │～S119
│          AND 3D CT IMAGE         │
└─────────────────────────────────┘
```

FIG. 4

FIG. 5

FIG. 6

FIG. 7

SEGMENTATION OF INDIVIDUAL TEETH

FIG. 8

801

802

803

CROWN SURFACE MATCHING

FIG. 9

S901

S903

SCAN DATA    CT DATA

MATCHING OF SCAN DATA AND CT DATA

3D KEY-POINT DETECTION

MATCHING

SCAN DATA TEETH CROWN SEGMENTATION

MATCHING OF SCAN DATA AND CT DATA

CROWN
SURFACE
MATCHING

S905

S907

## INTERNATIONAL SEARCH REPORT

| International application No. |
| --- |
| **PCT/KR2022/001042** |

**A.    CLASSIFICATION OF SUBJECT MATTER**

**G06T 7/33**(2017.01)i; **G06T 11/00**(2006.01)i; **G06T 15/20**(2011.01)i; **A61B 6/00**(2006.01)i; **A61B 6/14**(2006.01)i; **A61B 6/03**(2006.01)i; **A61B 5/00**(2006.01)i; **A61C 9/00**(2006.01)i

According to International Patent Classification (IPC) or to both national classification and IPC

**B.    FIELDS SEARCHED**

Minimum documentation searched (classification system followed by classification symbols)

G06T 7/33(2017.01); A61B 5/00(2006.01); A61B 6/03(2006.01); G06T 11/00(2006.01); G06T 3/60(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3차원 구강 스캔(3D oral scan), 컴퓨터 단층촬영(computed tomography, CT), 딥 러닝(deep learning), 치아 분할(tooth segmentation), 조화 함수(harmonic function), 히트맵(heatmap)

**C.    DOCUMENTS CONSIDERED TO BE RELEVANT**

| Category* | Citation of document, with indication, where appropriate, of the relevant passages | Relevant to claim No. |
| --- | --- | --- |
| Y | KR 10-2020-0120036 A (DIO CORPORATION) 21 October 2020 (2020-10-21) See paragraphs [0001], [0008], [0035]-[0036], [0047]-[0050], [0055], [0070]-[0074], [0087] and [0090]; and figures 3-4, 7, 9 and 11. | 1-3,5-6,8-16 |
| A | | 4,7 |
| Y | BARONE, Sandro et al. Creation of 3D Multi-Body Orthodontic Models by Using Independent Imaging Sensors. Sensors 2013. Volume 13, Issue 2, pp. 2033-2050, 05 February 2013. See section 2; and figure 1. | 1-3,5-6,8-16 |
| Y | LIAO, Sheng-Hui et al. Automatic Tooth Segmentation of Dental Mesh Based on Harmonic Fields. Hindawi Publishing Corporation. BioMed Research International. Volume 2015, pp. 1-10, 27 August 2015. See section 3; and figure 1. | 8-9 |
| A | KR 10-2018-0088061 A (EWOO SOFT CO., LTD. et al.) 03 August 2018 (2018-08-03) See paragraph [0011]. | 1-16 |

✓ Further documents are listed in the continuation of Box C.        ✓ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **04 May 2022** | **04 May 2022** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/001042**

**C.     DOCUMENTS CONSIDERED TO BE RELEVANT**

| Category* | Citation of document, with indication, where appropriate, of the relevant passages | Relevant to claim No. |
|---|---|---|
| A | KR 10-1953622 B1 (DIO CORPORATION) 23 May 2019 (2019-05-23)<br>See claim 1. | 1-16 |
| PX | KR 10-2273438 B1 (HDX WILL CORP.) 07 July 2021 (2021-07-07)<br>See claims 1 and 3-16.<br>* This document is the published patent of an earlier application that serves as a basis for claiming priority of the present international application. | 1-16 |

Form PCT/ISA/210 (second sheet) (July 2019)

## INTERNATIONAL SEARCH REPORT
### Information on patent family members

International application No.

**PCT/KR2022/001042**

| Patent document cited in search report | | | Publication date (day/month/year) | Patent family member(s) | | | Publication date (day/month/year) |
|---|---|---|---|---|---|---|---|
| KR | 10-2020-0120036 | A | 21 October 2020 | CN | 113645902 | A | 12 November 2021 |
| | | | | EP | 3954297 | A1 | 16 February 2022 |
| | | | | KR | 10-2020-0120035 | A | 21 October 2020 |
| | | | | KR | 10-2284623 | B1 | 02 August 2021 |
| | | | | KR | 10-2322634 | B1 | 08 November 2021 |
| | | | | WO | 2020-209496 | A1 | 15 October 2020 |
| KR | 10-2018-0088061 | A | 03 August 2018 | None | | | |
| KR | 10-1953622 | B1 | 23 May 2019 | None | | | |
| KR | 10-2273438 | B1 | 07 July 2021 | None | | | |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101911327